# EUROPEAN PATENT SPECIFICATION

(11) **EP 3 579 244 B1**
(45) Date of publication and mention of the grant of the patent: **21.10.2020**
(21) Application number: 18176780.7
(22) Date of filing: 08.06.2018
(51) Int. Cl.: G16H 40/63

(54) **METHOD FOR GENERATING A MOVEMENT SIGNAL OF AN OBJECT**
VERFAHREN ZUM ERZEUGEN EINES BEWEGUNGSSIGNALS EINES OBJEKTS
PROCÉDÉ PERMETTANT DE GÉNÉRER UN SIGNAL DE MOUVEMENT D'UN OBJET

(43) Date of publication of application: 11.12.2019
(73) Proprietor: Siemens Healthcare GmbH, 91052 Erlangen (DE)
(72) Inventor: Bacher, Mario, 90441 Nürnberg (DE); Speier, Peter, Dr., 91056 Erlangen (DE)

(56) References cited:
- Mario Bacher: "Cardiac Triggering Based on Locally Generated Pilot-Tones in a Commercial MRI Scanner: A Feasibility Study (Master Thesis)", , 25 October 2017 (2017-10-25), XP055519961, Retrieved from the Internet: URL:http://citenpl.internal.epo.org/wf/sto rage/166AEE29EAF0009E6DA/originalPdf [retrieved on 2018-10-30]
- VINCENT WU ET AL: "Adaptive noise cancellation to suppress electrocardiography artifacts during real-time interventional MRI", JOURNAL OF MAGNETIC RESONANCE IMAGING, vol. 33, no. 5, 20 April 2011 (2011-04-20) , pages 1184-1193, XP055437236, US ISSN: 1053-1807, DOI: 10.1002/jmri.22530
- YURONG LUO ET AL: "A Hierarchical Method for Removal of Baseline Drift from Biomedical Signals: Application in ECG Analysis", THE SCIENTIFIC WORLD JOURNAL, vol. 2013, 1 January 2013 (2013-01-01), pages 1-10, XP055519977, DOI: 10.1155/2013/896056
- I Graesslin ET AL: "Advancements in Contact-free Respiration Monitoring using RF Pick-up coils", Proceedings of the International Society for Magnetic resonance in medicine, 1 January 2010 (2010-01-01), page 3045, XP055519013, United States DOI: 10.1002/mrm.26866 Retrieved from the Internet: URL:https://cds.ismrm.org/protected/10MPro ceedings/files/3045_4702.pdf [retrieved on 2018-10-25]
- Lea Schroeder ET AL: "A Novel Method for Contact-Free Cardiac Synchronization Using the Pilot Tone Navigator", Proceedings of the International Society for Magnetic Resonance in Medicine, 1 January 2016 (2016-01-01), pages 416-418, XP055519012, United States DOI: 10.1002/mrm.27038 Retrieved from the Internet: URL:https://pdfs.semanticscholar.org/b1ab/ eb7c4b03d7ce1fa3f557666598abfd5c4a36.pdf [retrieved on 2018-10-25]

## Description

The present invention relates to a method for generating a movement signal of an object, in particular a body part of a human or animal, to a method for generating a medical image data set of an object undergoing a movement, to a medical imaging device configured for acquiring medical image data sets with reduced motion artefacts, and to a computer program product.

Many medical imaging modalities, such as computer tomography (CT), magnetic resonance imaging (MRI) or Positron emission tomography (PET) are sensitive to patient motion during image acquisition. For example, high quality and high coverage MRI measurements typically take several minutes. During this time, motion inside the imaged volume will degrade image quality and make it non-diagnostic, unless the motion is tracked and taken into account during reconstruction. Apart from motion in non-compliant patients, the unavoidable respiratory motion and cardiac motion needs to be taken into account.

Exemplary means and methods for motion correction are described in US 20170160367 A1, US 20160313432 A1, US 20100142789 A1, US 20140355855 A1 and US 7205763 B2.

According to the prior art, motion artefacts in medical images are reduced by triggering the image acquisition to extreme motion states, like end-inspiration or end-expiration. This suffices if the respiratory motion is regular. However, in clinical practice, respiratory patterns, e.g. the position of end-inspiration, often change during an examination. To achieve robust motion synchronisation, therefore quantitative navigation methods are desired, e.g., methods providing more information about the motion than the times of the extreme motion states.

An example of quantitative navigation in MRI is respiratory gating using liver dome MR navigators. Here, a specific navigator value corresponds to one geometric respiratory phase for the whole scan. Thus, the method assumes that respiration can be described by a single parameter, for example the position of the upper side of the liver.

Electromagnetic navigation (EMN) methods like the pilot tone (PT) method or the Biomatrix respiratory sensor have recently been investigated for providing motion information. However, these methods tend to be affected by signal drifts. This is because the modulation depth due to the motion to be observed is at best of the order a few percent. Thus, minor drifts in the movement signal, for example caused by minor gain changes in receive path electronics, create large drifts in the net motion signal.

The pilot tone navigation method has first been described, for example in Lea Schröder, Jens Wetzel, Andreas Maier, Lars Lauer, Jan Bollenbeck, Matthias Fenchel and Peter Speier: "A Novel Method for Contact-Free Cardiac Synchronisation Using the Pilot Tone Navigator", Proceedings of the International Society for Magnetic Resonance in Medicine (ISMRM), 2016, p. 416. The pilot tone method is further described in DE 10 2015 203 385 A1 and DE 10 2015 224 162 A1, which describes a way of extracting the various signal components from a multi-channel pilot tone signal by means of a principal component analysis and/or independent component analysis.

Another electromagnetic navigation method is known, for example from E. Graesslin, G. Mends, A. Guillaume at al. "Advancements in Contact-Free Respiration Monitoring using RF Pick-up coils", Proceedings of ISMRM (2010), p. 3045. This EMN method relies on monitoring the complex currents in the transmit RF-coils used during magnetic resonance imaging via Pick-up coils. Short rectangular monitoring pulses are integrated into the pulse sequence used for imaging. The drift of the RF-amplifiers is seen in the Pick-up coils signals, and corrected by means of magnitude correction of the Pick-up coils signals with the forward power measured between amplifier and circulator output. However, this drift correction requires further measurements in the receive path electronics.

Mario Bacher: "Cardiac Triggering Based on Locally Generated Pilot-Tones in a Commercial MRI Scanner: A feasibility Study (Master Thesis), October 25, 2017, Graz University of Technology, discloses a method of generating a Pilot Tone (PT) signal by a low-cost local RF transmitter and receiving the PT signal by the standard MR coils. A standard four-lead electrocardiogram (ECG) and ECG R-peak positions were recorded alongside as ground truth. The peak-to-peak modulation amplitude M was extracted and used to quantify the strength of modulation by the modulation depth in percent.

Accordingly, it is an object of the invention to provide a method and medical imaging device capable of providing quantitative motion information in order to acquire medical images with reduced motion artefacts.

It is a further object of the invention to provide a novel way of characterising and compensating a drift in the navigation signal in EMN.

The above object is achieved by the methods according to claim 1 for generating a movement signal of an object, by the method of claim 11 for generating a medical image data set of the object, as well as by the medical imaging device of claim 14 and the computer program product of claim 15. All features, embodiments and advantages described with regard to one aspect or claim category of the invention are also applicable on all other aspects and categories. Preferred embodiments and features are set out in the dependent claims, as well as in the description and figures.

According to a first aspect of the present invention, a method for generating a movement signal of an object is provided, in particular of a body part of a human or animal, wherein the movement signal provides quantitative information on a movement of the object, the method comprising the following steps:
a) Acquiring a navigation signal from the object, wherein the navigation signal is an electromagnetic signal, which is modulated by movements of the object;
b) Extracting a reference signal from the navigation signal;
c) Determining a parameter having a known time-dependency from the reference signal;
d) Correcting the navigation signal based on the parameter or the time-average of said parameter in order to reduce a signal drift in the navigation signal;
e) Extracting the movement signal from the navigation signal.
The invention has recognized that the signal drift in EMN is caused by changes in coil sensitivity, receiver gain and signal routing attenuation. The most likely cause for these changes is the heating of the components, e.g. due to RF and gradient activity of a magnetic resonance scanner. Therefore, the signal drift is relatively slow compared to physiologic movements, such as cardiac and respiratory motion, and this opens up the possibility of using signal components having a known time-dependency, e.g. signal components related to quasi-stationary or quasi-periodic physiologic movements, for correction. The terms "motion" and "movement" are used interchangeably herein.

The invention involves extracting a reference signal from the navigation signal, and determining a parameter having a known time-dependency from the reference signal. For example, said parameter may be constant in time, at least when time-averaged, e.g. over 1 to 120, preferably 5-30 seconds. Such parameter is preferably extracted from a known characteristic of the reference signal, for example a quasi-periodic behaviour, in particular the parameter characterizes a quasi-periodic time behaviour of the reference signal. Thus, since the expected time dependency of the parameter is known, any difference between the actually observed time dependency and the known (expected) time dependency must be due to the signal drift. Therefore, a measure for the signal drift can be determined during an examination of the human or animal, and can be used to correct the signal drift in the movement signal. Thus, the parameter serves as a gain reference value. Since the reference signal and the movement signal are both extracted from the same navigation signal, they will be subjected at least approximately to the same signal drift.

In case a parameter or time-averaged parameter which is constant over time is determined from the reference signal, the movement signal can for example be multiplied with the normalized inverse of said parameter in the correction. Preferably, the parameter is related to the signal amplitude. It may, for example be the absolute peak-to-peak amplitude, in case the reference signal or a parameter derived from the reference signal, e.g. peak-to-peak amplitude is a signal expected to be constant over time, or at least constant when averaged over a time interval which is short enough to follow the drift. In useful embodiments, the parameter is determined repeatedly, for example in pre-determined time intervals, and the movement signal is continuously corrected using the latest calculated parameter. The parameter may be any parameter that can be calculated from the reference signal, e.g. the absolute signal size of the signal, or its slope, or the amplitude of an oscillation in the reference signal. If such parameters are known to be constant over time, any drift in these parameters must be due to unwanted signal drifts in the navigation signal, and thus, can be used for correction.

The corrected movement signal is preferably used for navigating a medical examination, in particular a medical image acquisition or scan from the body part going on at the same time, i.e. while the navigation signal is acquired. The image acquisition may be by any known medical imaging modality, such as CT, SPECT, PET, ultrasound, but is preferably by MRI, since MRI already provides the equipment for measuring an EMN signal. The body part may by any body part of which diagnostic images are to be acquired, e.g., head, chest, abdomen, shoulder, hip, or part of the extremities such as arms and legs. The image acquisition may be synchronised or gated by the drift-corrected movement signal.

The navigation signal can be any electromagnetic navigation (EMN) signal, which is modulated by movement of the object, and thus able to detect movement in a part of the human or animal body, in particular allowing the detection of respiratory and cardiac movement, and suffering from gain drift. In particular, the navigation signal is an EMN signal, in which the absolute signal size (and not only the frequency or phase of the signal) is modulated by movements of the object.

In useful embodiments, the navigation signal is an electromagnetic signal comprising several signal components, wherein at least some of the signal components correspond to different, independent movements of the object, for example the various physiologic motions such as respiration and heartbeat. The navigation signal may be acquired from a probe close to the part of the body from which the movement signal is to be generated, wherein the probe may be a coil, e.g., a local RF coil, an observation circuit or other electromagnetic sensor. In useful embodiments, the probe has several individual detectors, such as the various coils of multi-channel RF-coils, e.g., an array coil. In useful embodiments, the various channels deliver linear mixtures of the individual components of the navigation signal. By principal component analysis (PCA) and/or independent component analysis (ICA), the various signal components having different characteristics in the time- and/or frequency-domain, in particular the signal components corresponding to the different physiologic movements, can be extracted from the navigation signal.

The navigation signal may be either pulsed or continuous, e.g., a continuous-wave signal. It may be modulated by movements of the object via various mechanisms, for example in that it detects a reflection of an electromagnetic signal in the object, or in measuring the gain, the efficiency, the degree of tuning/detuning, the phase or the loading of a coil placed close to the human or animal body, in particular a radio frequency (RF) coil. The navigation signal may, for example be acquired from a biomatrix respiratory sensor, which is based on an oscillator circuit, e.g. built into the patient table of a medical imaging scanner. The loading of said oscillator circuit is measured continuously during a medical imaging examination, thereby generating a navigation signal which can be used in the inventive method. In another embodiment, the navigation signal is a pulsed signal and is measured by pick-up coils on the transmit RF coil, for example as described in E. Graesslin, G. Mends, A. Guillaume at al. "Advancements in Contact-Free Respiration Monitoring using RF Pick-up coils", Proceedings of ISMRM (2010), p. 3045. In a further embodiment, the navigation signal is a Pilot Tone signal, as described in more detail below.

For all these techniques, a drift correction is important, since the movement one desires to detect, such as cardiac and respiratory movement, modulates the signal amplitude only to a small degree, for example up to 1 to 5 percent. However, if one wants to derive not just a movement signal allowing the detection of extreme motion states (such as the time points of end-systole and end-diastole), it is important that the signal drift in the navigation signal is corrected, since otherwise any signal drift will falsify the detected movement state. Especially for respiratory movement, it is quite common that the extreme motion states of a particular human subject or patient will change over an examination: In the beginning, the patient may be nervous and breathe shallowly.

During the examination, he might relax, and the breathing is deepened. Thus, the positions of the organs at the extreme motion states (end-expiration and end-inspiration) will change considerably. By detecting only the time points of such extreme motion states, and assuming their positions to remain constant, a good motion correction is not possible.

The reference signal is preferably a signal component of the navigation signal, for example a signal component having a certain time-dependency known a-priori. In useful embodiments, the reference signal corresponds to a physiologic movement of the object. Further, it preferably has a quasi-periodic time behaviour. "Quasi-periodic" means that the movement cycles are not exact, neither in frequency nor phase, such as the physiologic motions such as heartbeat and respiration. The reference signal can be extracted for example by means of a frequency analysis, if its expected frequency is within a certain known range. For example, the cardiac signal has a known quasi-periodic time-dependency and can therefore be extracted by a frequency analysis. In case the reference signal is recorded on several channels, the various signal components can be extracted by means of principal component analysis (PCA) and/or independent component analysis (ICA) or other blind and/or semi-blind source-separation techniques. Blind/semi-blind methods generally deal with the problem of extracting components of a signal mixture with no (blind) or limited (semiblind) a-priori knowledge of the actual sources. An alterative to ICA is e.g. the SOBI algorithm.

From this reference signal, a parameter having a known time-dependency is determined, wherein the known time-dependency may also be that the parameter is constant or at least its time-average is constant. Accordingly, in useful embodiments a reference signal is identified in the pilot tone data, whose amplitude behaviour is known and independent of the drift. From this amplitude behaviour, a parameter having a known time-dependency is determined, for example a constant parameter such as the amplitude of a periodic or quasi-periodic oscillation in the reference signal, or the absolute signal size. In case the parameter is expected to be constant, the navigation signal may be multiplied with the inverse of the parameter in order to reduce and ideally eliminate signal drift.

Finally, the desired movement signal providing quantitative information on a movement of the object is extracted from the navigation signal, again for example by frequency analysis, in case the movement has a frequency in a known range, or by PCA and/or ICA. Preferably, also the desired movement signal corresponds to a physiologic movement of the object, in particular a quasi-periodic movement. Since such a movement signal is extracted from the corrected navigation signal, its drift will be minimised, and thus it will provide quantitative information on said movement of the object, wherein such movement may be any movement - respiratory, cardiac or voluntary patient movement. Accordingly, the corrected movement signal may be used for synchronising the acquisition of a medical image data set, or in retrospective gating of a medical image data set acquired while the inventive method was carried out, in order to reduce undesired motion artefacts in the medical image data set.

According to a useful embodiment, the reference signal has a quasi-periodic time-dependency, and the parameter is the amplitude of said quasi-periodic signal. For example, the reference signal may be caused by a quasi-periodic physiologic motion such as the heartbeat. This choice is advantageous because a quasi-periodic signal component can be easily extracted from the navigation signal, and the amplitude of a periodic curve can easily be determined.

According to a useful embodiment, the known time-dependency of the parameter determined from the reference signal is that the parameter is constant over time, or at least the time-average of said parameter is constant over time. Thereby, the parameter may serve as a measure for signal drift. The invention has recognized that the signal drifts in the navigation signal can be large compared to the physiologic motion signals, but are usually very slow compared to the physiologic motions, and typically evolve over several tens of seconds to minutes. Therefore, it is possible to average drift characterisation data, such as the parameter extracted from the reference signal, over relatively long time periods in order to increase its accuracy. The average time period may, for example be 0.5 - 100 seconds, more preferred 1 - 20 seconds.

According to a preferred embodiment, the reference signal is the cardiac signal component, or cardiac trace, i.e,. a signal related to the heart movement of the body of the human or animal. In a useful embodiment, the parameter extracted therefrom is the peak-to-peak amplitude between the point of maximum (end of systolic phase) and minimal (end of diastolic phase) contraction. The cardiac signal component is particularly suitable, because it consists of a quasi-periodic curve with the special property that the peak-to-peak amplitude is nearly constant over time, especially when averaged over several cycles of heart movement or heartbeats, for example over 5 - 50, preferably 10 - 20 heartbeats. The reason is that the heart of a human (when at rest) tends to have a relatively constant stroke volume, when averaged over several heartbeats, even if irregularities in the frequency are common. Therefore, the amplitude of the cardiac signal component, e.g. the peak-to-peak amplitude, can serve, after sufficient averaging, as a measure of the receiver gain. Thus, it can be used to correct the gain, in particular for each channel. Interestingly, the cardiac signal is likely to be visible not only if the body part is the heart, but also in all other parts of the body, in particular the head, abdomen and even the extremities, due to pulsatile flow expanding the blood vessels in all parts of the body.

In practice, the cardiac signal component can still be contaminated with respiratory residues. However, these signal contributions can be separated before the analysis, e.g. before determining the parameter (e.g. peak-to-peak amplitude) based on the lower frequency content.

According to a useful embodiment, the navigation signal is acquired continuously over the duration of the medical examination or scan, and the parameter or the time-average of said parameter of the reference signal is determined repeatedly during the medical examination. This allows continued correction of the signal drift. Thus, the method is preferably performed during a medical examination, preferably a medical imaging procedure on said body part. The invention is applicable in magnetic resonance imaging, but may also be used during a CT, X-ray, PET or single photon emission computer tomography (SPECT) examination, thus any medical imaging procedure having acquisition times longer than one breath-hold.

In case the parameter used as gain reference value is related to a quasi-periodic time-dependency of the reference signal, the parameter may be determined at each period of the quasi-periodic signal, preferably it is averaged over several periods, and the following portion of the navigation signal is corrected based on this parameter. In the next periods of the reference signal, a new time-average parameter is determined, and then the new parameter is used for the correction. Thereby, the parameter or time-averaged parameter is determined repeatedly during the medical examination. In other embodiments, the parameter is determined afresh again in specified time intervals, for example every 1 - 3 minutes, and used for correction during the next time interval.

According to a useful embodiment, the parameter is normalized. Thus, when the parameter is first determined, it is set to the value one. When the parameter (or the time-averaged parameter) is determined again, the same normalisation factor is used, so that any variation or drift in the parameter will become apparent in that the parameter does not have the value of one any more. Thereby, the calculation of the corrected movement signal is simplified.

According to useful embodiment, the movement signal finally extracted from the navigation signal provides quantitative information on a quasi-periodic physiologic movement of the human or animal, preferably on a respiratory movement. This application of the invention is particularly advantageous, since the respiratory movement is relatively great, causing displacements by several centimetres. On the other hand, as mentioned before, it is not particularly regular, and various breaths during examination may be deep or shallow. Accordingly, it is important to have quantitative information, e.g., a movement signal the curve of which is directly related to the position of the moving body part at each time point, for example by a linear correlation. Thereby, it is possible to determine exactly the position of each organ on an image acquired by the medical examination.

According to a preferred embodiment, the navigation signal is acquired on several signal channels, in particular by a multi-channel RF coil for magnetic resonance imaging. Thereby, the navigation signal has several signal channels. By combining the various channels in a certain way, the different signal components having specific characteristics may be extracted, such as the cardiac signal component and the respiratory signal component. In useful embodiments, the channel combination coefficients for each desired signal component is determined from a calibration portion or calibration phase of the movement signal, acquired e.g. during 1 to 60 seconds before or at the beginning of a medical examination, e.g. by PCA and/or ICA, whereby a demixing matrix is determined, as described below.

The correction does not change the channel combination coefficients significantly because gain variations are slow and small (within a few percent) and the modulation depth of the navigation signal, in particular a pilot tone signal due to the much faster physiologic processes, i.e., the net navigation signal, is on the order of a few percent as well. Therefore, the first order effect of a channel-wise gain variation is a slowly varying offset in the channel combined pilot tone component. Therefore, instead of applying the correction for each channel individually, it can be applied to the combined signal instead, i.e. to the extractive movement signal.

Thus, in a useful embodiment, steps d) and e) of the inventive method may be interchanged: Thus, the movement signal is first extracted from the uncorrected navigation signal. The correction based on the parameter or the time-average of said parameter is then carried out on the extracted movement signal, in order to compensate a signal drift in the movements signal. Naturally, if the movement signal is a linear combination of the various signal components or channels of the navigation signal, it makes no difference whether the correction is applied to each channel individually, or to the combined signal, i.e. the extracted movement signal, instead.

According to a preferred embodiment, the reference signal and the movement signal (also called reference signal component and movement signal component) are extracted from the navigation signal by an PCA, ICA and/or a frequency analysis. This is based on the fact that the various signal components have different characteristics, for example different frequencies that are independent from one another. Therefore, the various signal components can be separated from each other.

According to a preferred embodiment, the navigation signal is a pilot tone (PT) signal, wherein the pilot tone signal is generated by: emitting a radio frequency (RF) signal outside the bandwidth of an MR signal acquired during magnetic resonance imaging of the part of the human or animal body, and recording the radio frequency signal, modulated by a movement of the human or animal body, by an RF coil, in particular a multi-channel RF coil for magnetic resonance imaging, wherein the recorded radio frequency signal is the navigation signal.

Preferably, the RF signal is a coherent or continuous frequency signal generated by an independent continuous-wave RF source outside the receive bandwidth of the actually scanned MR field of view, but within the range of the oversampling bandwidth acquired during every readout. The RF signal is usually not pulsed, but at least quasi-continuous. Therefore, its frequency bandwidth is very narrow and can be selected outside the bandwidth of a MR signal acquired during MR imaging, e.g. about 20 to 200 kHz away from the centre frequency of the RF coil.

The pilot tone (PT) is particularly useful in that it is outside the receive bandwidth of the magnetic resonance image, and therefore does not interfere with the magnetic resonance (MR) signal. Further, the pilot tone signal is acquired independent of the imaging sequence and can therefore be used with arbitrary imaging sequences.

A method for reliably extracting the cardiac movement signal from the PT signal is described in EP 17179814.3, and the method is also applicable for extracting the respiratory movement signal. In particular, this application describes steps of
a. Providing a Pilot Tone signal acquired from the body part by a magnetic resonance receiver coil arrangement comprising several channels, wherein the Pilot Tone signal comprises several signal components associated with the several channels;
b. From a calibration portion of the Pilot Tone signal , calculating a demixing matrix by means of an Independent Component Analysis (ICA) algorithm, wherein the demixing matrix calculates the independent components from the several signal components,
c. Selecting the independent component(s) corresponding to at least one particular movement type, in particular the cardiac movement,
d. Applying the demixing matrix to the further portions of the Pilot Tone signal to obtain at least one movement signal representing one particular movement type, in particular the cardiac movement.

The description and examples of these steps in EP 17179814.3 may be also used in the present invention.

The "calibration portion" of the Pilot Tone signal is preferably a short portion covering only a few (e.g. 1 to 20) heart beats acquired before or at the beginning of the scan of medical data. From this calibration portion, a demixing matrix is determined, e.g. by first determining the principle components through PCA, and then identifying the physiologic components by means of ICA. Prior to the calculation of the demixing matrix, the Pilot Tone signal may be (pre-)processed, in particular by down-sampling to a new sampling frequency that is sufficient to capture cardiac dynamics, preferably to 50 to 300 Hz. To avoid aliasing of high-frequency noise, the signal is preferably low pass filtered prior to down sampling. In preferred embodiments, the phases of all channels are then normalized to a reference phase of a selected channel and only relative phase offsets to this reference are further considered. In order to reduce the complexity of the ICA problem, further pre-processing steps are preferably performed before the calculation of the demixing matrix, or before the ICA algorithm. These may include centering (subtracting the mean so that the resulting signals are zero-mean), whitening/sphering to ensure that all signals have unit variance and are uncorrelated, i.e. for a given (n x m) matrix x, where n is the number of samples and m is the number of channels, Cov(x) = E{xxT}=I, with I the identity matrix, and dimensionality reduction.

After such pre-processing, ICA is used to extract several independent components from the calibration portion of the Pilot Tone signal. Once the independent component(s) corresponding to the desired movement types have been selected, a demixing matrix or vector that extracts these movement type(s) is applied to the further portions of the Pilot Tone signal, preferably in real time. When several movement types are to be extracted, applying the demixing matrix results in several movement signals, each representing one particular movement type.

According to a useful embodiment of the invention, a pilot tone signal component of a physiological signal with a known characteristic, such as the constant amplitude of the quasi-periodic cardiac trace, is used as gain reference. This enables to compensate drifts in the receive gain of the individual channels of the electromagnetic navigation signal. Thereby, it is possible to extract quantitative movement information from the navigation signal, for example on the respiratory movement state.

The invention is also directed to a method for generating a medical image data set of an object undergoing a movement, in particular a part of the human or animal body, e.g. any body part of which images are to be acquired, e.g. the head, the chest, the abdomen, the shoulder, the hip or any extremity. Placing the object in the medical imaging device means that e.g. a human subject or patient is placed on a patient table and shifted into the sensitive region of the medical imaging device, for example the bore of an MR scanner.

The movement signal is used during or after image acquisition, either for synchronizing the MR image sequence to the movement signal, in particular to the respiratory movement of the part of the human or animal body, or for retrospectively gating the MR image data acquired, so that medical images may be reconstructed without motion artefacts. By "synchronizing or gating" all methods are meant which use information on the movement state of an object, especially an object undergoing a quasi-periodic movement, to record an image without or at least reduced motion artefacts. This can be done e.g. by synchronizing or timing the acquisition such that image date acquisition takes place at the desired motion states. These can be either at times when there is no motion, or care is taken to a acquire data at specific motion states of the object, so that the image data set includes a time-series of images which can be viewed in sequence to analyse the movement (cine-mode). By "gating", it is meant that the present movement state is recorded together with the image data recorded at the time, so that the data can be reconstructed retrospectively by rearranging the data correctly, i.e. all image data corresponding to one movement state are reconstructed into one image.

The image data set can be a two-dimensional (2D), free-dimensional (3D) or four-dimensional (4D) image data set, wherein the fourth dimension is time. Usually, such image data set includes an array of data points, e.g. including grey level information, and usually a header containing information on the time of image. The image data set may, for example be in DICOM standard.

The invention is also directed to a medical imaging device, in particular a magnetic resonance imaging device (MR scanner) adapted to perform the inventive method. The MR scanner will comprise a magnet for generating the main magnetic field, gradient coils, as well as an RF coil for acquiring the navigation signal from the object. In case of pilot tone navigation, the pilot tone signal is usually emitted by an external frequency source which is placed into the bore of the main magnet. However, it may also be emitted by the body coil. The medical imaging device may also be an ultra sound device, PET, SPECT or CT device.

The invention is also directed to a computer program or computer program product comprising software code portions which induce a processor, in particular a processor controlling a medical imaging device such as an MR scanner, to perform the inventive method, when the software code portions are executed on the processor. The processor may be part of a workstation related to a medical imaging device or it may be part of a standalone computer, cloud computer, server, tablet computer, any mobile or portable device such as a laptop.

According to a further aspect of the invention the invention is directed to a digital storage medium on which the inventive computer program or computer program product is stored. The digital storage medium can be part of a computer, such as a hard disc, solid state disc, etc., wherein the computer may also include the processor describe herein. Further, the digital storage medium may be a cloud storage, the storage of a medical imaging device, or any kind of portable storage such as a USB-stick, CD-ROM, SD-card, etc.

The invention shall now be described by means of non-limiting examples with reference to the attached drawings, in which:
- Fig. 1: shows a magnetic resonance scanner according to an embodiment of the invention in a schematic view;
- Fig. 2: shows a graph of an example pilot tone signal, as well as of a respiratory signal component and cardiac signal component extracted therefrom, in signal amplitude in arbitrary units versus time in seconds;
- Fig. 3: shows a graph of a filtered cardiac signal component during one heart cycle;
- Fig. 4: shows graphs of a simulated respiratory signal (top) and simulated cardiac signal (bottom), in signal amplitude in arbitrary units versus time in seconds;
- Fig. 5: shows a schematic flow diagram of an embodiment of the inventive method.

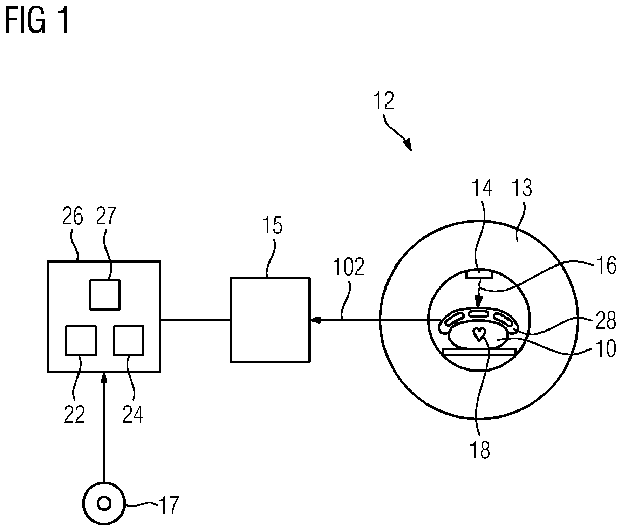

With reference to Fig. 1, an MR scanner 12 includes a main magnet 13, with a human subject 10 or patient placed in its bore. A receiver coil arrangement 28, for example a local coil such as a head-coil or chest-array-coil, is placed close to the body part from which images are to be acquired. The human subject 10 is subject to various movements, especially the two physiologic, quasi-periodic movements of respiratory motion and the motion of the heart 18. The MR-scanner 12 includes further components not shown herein, such as gradient coils for generating gradient fields, as well as usually a body coil, which may be used as RF transmitter for transmitting the RF-pulses for MR image acquisition, and possibly the radio frequency signal outside the MR receive bandwidth, used for generating the pilot tone signal 16. In the embodiment shown, the pilot tone signal 16 is emitted by a separate RF source 14, which is positioned inside the bore of the main magnet 13. Preferably, such separate RF source 14 may be strapped to the local coil 28. The pilot tone signal 16 is modulated by the movement of the heart 18 and further movements of the human subject 10. The modulated pilot tone signal together with the MR signal 102 is received by the receiver coil arrangement 28 and is further transmitted to a receiver 15, which includes electronic components as known in the art, for example a pre-amplifier and amplifier, as well as other electronic components such as filters. The receiver 15 is a likely contributor to gain changes creating large drifts in the net pilot tone signal. The receiver 15 is connected to a computer device 26 including a control unit 24, which controls the activity of the MR-scanner 12, in particular the image data acquisition. The MR signal 102 (including the PT signal) is received and amplified by the receiver 15 and further processed in the control unit 24, which may include a processor, and/or the computer device 26. The computer device 26 can also include a digital storage medium 22 and a user-interface 27 including e.g. a display, a keyboard, a mouse, touchscreen or such like. A compact disk 17 may include a computer program which, when loaded into the computer device 26, configures the MR-scanner 12 to perform a method according to the invention.

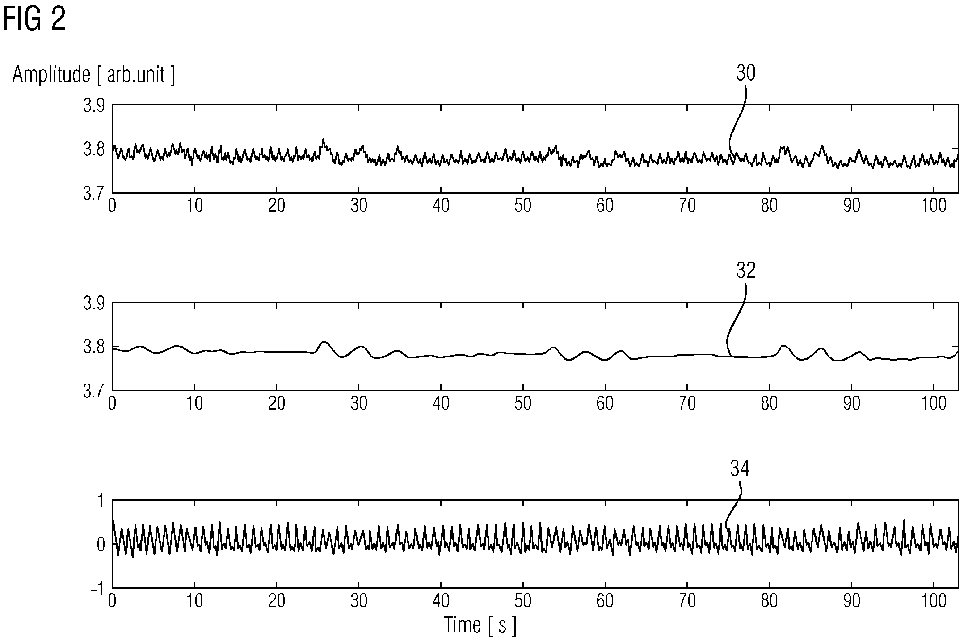

Fig. 2 illustrates the various signal components of the pilot tone signal, also referred to as navigation signal: The navigation signal is preferably acquired on several channels, wherein each channel may correspond to one coil element of the RF coil 28. Thus, the navigation signal comprises several signal components or channels. The upper trace 30 shows one channel of the navigation signal. One can see that this signal 30 is a super position of various signal elements having different characteristics and frequencies. These various signal components can be extracted from the navigation signal by means of frequency analysis, or in the case of a signal having several signal channels, by PCA and/or ICA. Thereby, the various signal components can be extracted. For example, trace 32 shows a respiratory signal acquired during instructed breathing: The subject is told to take three deep breaths, interrupted by a breath hold. Trace 32 demonstrates very well the fact that the respiratory movement - although approximately periodic (quasi-periodic) - is highly irregular: some breaths are deep, others are shallow. Therefore, if one wants to extract quantitative information, for example from the absolute signal size or amplitude of the respiratory signal, one needs to ensure that there is no drift.

Trace 34 shows a cardiac signal extracted from the navigation signal 30, and which shows a distinct periodic behaviour with the frequency of about 1s⁻¹ corresponding to the heartbeat. The amplitude of the oscillation of the cardiac signal 34 is variable from heartbeat to heartbeat, but is roughly constant when averaged over e.g. more than 10 heartbeats. Thus, it may be used, after sufficient averaging, as a measure of the receiver gain for each channel and can be used to correct the gain for each channel.

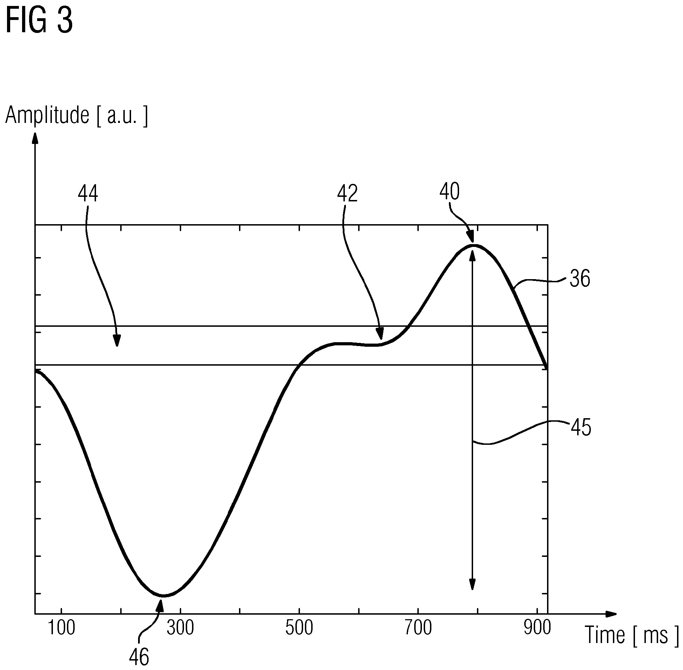

The cardiac signal component 36 of one cardiac cycle is shown in magnification in fig. 3. The cardiac signal component 36, also referred to as cardiac trace, shown here has been filtered, e.g. by a switched Kalman filter based on a model generated by analysis of the cardiac component acquired during a calibration phase. This filtered cardiac trace 36 is shown in a plot of amplitude in arbitrary units versus time. From the filtered cardiac trace 36, one can determine the following points of interest: The minimum 46 of the cardiac component trace 36 indicates end-systole, i.e. the maximum contraction and resting phase. The maximum 40 of the cardiac component indicates end-diastole i.e. the physiological phase of maximum expansion of the heart during the resting phase. The plateau 42 can be associated with the mid-diastolic phase, in which the ventricle is relaxed, i.e. a resting phase. The area 44 indicates the signal level for R-wave occurrence, and may be used in a threshold trigger. From the cardiac trace 36 also the first and/or second derivative can be derived. The minimum of the first derivative of the cardiac trace indicates times of maximum velocity. The difference in amplitude between minimum 46 and maximum 40 is the signal difference 45 between the diastolic and the systolic phase of the heart, and can be used as a gain reference, especially when averaged over several heart-cycles. For example, the respiratory signal component 32 can be divided by the averaged signal difference 45, so that any signal drift occurring in both signals is eliminated.

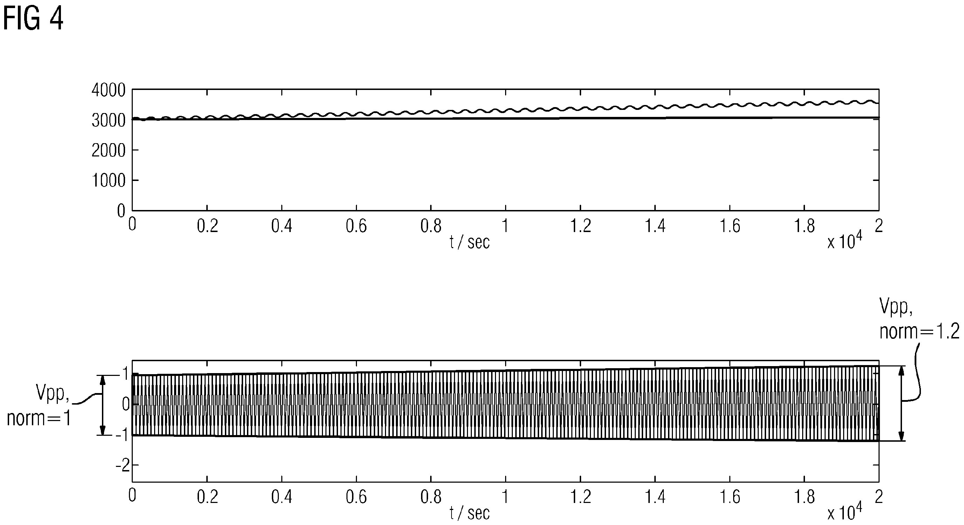

Fig. 4 shows simulated respiratory (top) and cardiac (bottom) signals, in order to demonstrate the effect of the gain drift: In the simulated respiratory signal in the top graph, the offset (i.e. the absolute signal amplitude) is large (3000a.u.) compared to the respiratory signal having a modulation depth of about 1.6%. An assumed slow, linear gain drift from gain=1 to gain=1.2 is simulated. Since the absolute amplitude of the respiratory signal is much higher than the modulation depth of the motion signal, gain variation presents primarily as drift. The bottom graph shows a simulated cardiac trace, in which the offset (5000 a.u.) has been removed for better visibility. Since we can assume the peak-to-peak amplitude to be constant over time, any change in observed peak-to-peak signal amplitude Vpp must be due to gain changes. Thus, the gain variation can be approximated by continuously measuring the peak-to-peak amplitude. Vpp is normalized at the beginning to Vpp,norm = 1 to allow for simple division of the e.g. respiratory signal with Vpp,norm. At the end of the observed time period, Vpp,norm has risen from 1.0 to 1.2.

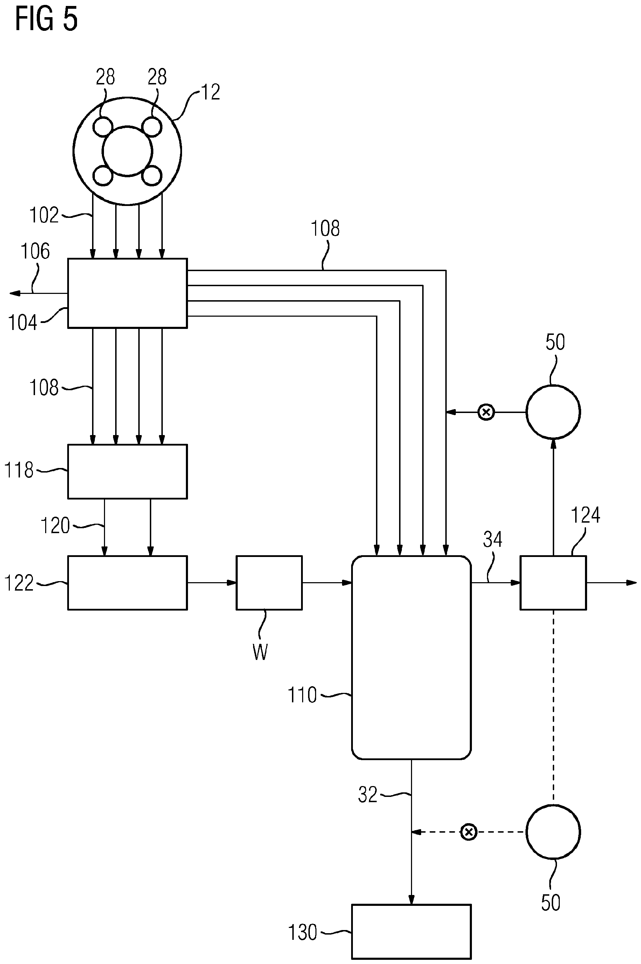

Fig. 5 gives an overview of the method according to the invention. The MR-scanner 12 includes a receiver coil arrangement 28 with (in this schematic example) four coils/channels. When the acquisition of the navigation signal and MR signal starts, the RF coil acquires a signal 102 having four signal components. In a separation step 104, the navigation signal 108, namely a complex pilot tone signal, is separated from the MR data 106. The MR data 106 is further possessed to produce MR image data, as known in the art. In step 104, the absolute frequency of the navigation signal may be determined.

The pilot tone signal 108 comprising the four channels or signal components is optionally subjected to further processing steps, such as pre-processing by low-pass or bandpass filtering and centering. The pre-processed signal may further be subjected to a normalization step, in which the phases of all channels are normalized to a reference phase.

The optionally normalized, complex pilot tone signals 108 are then further processed in step 118 to separate the various movement types, by which the pilot tone signal is modulated. According to a useful embodiment, this is done first by principle component analysis, in which the largest principle components are extracted. These are then subjected to independent component analysis (ICA). Through the ICA, the different signal components 120 corresponding to the different movement types are separated. Thereby, particularly a reduction in dimensionality occurs, i.e. four components 108 are reduced to two components 120 representing respiratory motion and cardiac motion. To identify the cardiac signal component from the independent components 120, e.g. the signal energy in the cardiac motion band can be calculated for each independent component, compared to the signal energy in other frequency bands, and the signal component with the highest relative signal energy in frequency band corresponding to cardiac motion is selected. Alternatively, the degree of correlation of each signal component with a typical cardiac trace may be calculated. Similarly, the single component corresponding to the respiratory movement may be calculated. Once the independent component have been correctly identified, a demixing matrix W can be calculated, preferably automatically. The demixing matrix W may correspond to a linear combination of the signal components 108 of the several receiver channels 28.

Steps 118 and 122 are preferably carried out on a calibration portion of the navigation signal at the beginning of the medical examination. During the calibration phase, the coil combination corresponding to the reference signal (cardiac signal component) is detected by the usual processes, e.g. ICA or frequency analysis.

The demixing matrix W is then stored and used during the main part of the medical examination. In particular, the demixing matrix W is applied to the incoming further pilot tone signal 108, which is multiplied with the demixing matrix W in step 110 to obtain at least one selected independent component, in particular the cardiac signal component 34 and the respiratory signal component 32. The cardiac trace 34 is analysed in step 124, either continuously or in suitable intervals during the image acquisition. In step 124, the signal difference 45 between the diastolic and systolic phase is determined and averaged over several cycles of heart movement. This is used as the gain reference parameter 50, which may be used to correct the navigation signal in two alternative ways: As shown in unbroken lines, the gain reference parameter 50 may be used to correct the pilot tone signal 108, i.e. the uncombined pilot tone data before applying the demixing matrix in step 110, e.g. by multiplying with its inverse. Alternatively, as shown in dashed lines, instead of applying the correction for each channel individually, it can be applied to the combined respiratory signal component 32 instead, i.e. after the movement signal has been extracted from the navigation signal. In either case, the demixing matrix W is also applied to the (corrected or uncorrected) pilot tone signal 108 in step 110, in order to separate the respiratory signal component 32, which may be used as a movement signal to synchronize the MR acquisition in real time in step 130. In useful embodiments, the reference value 50 is updated e.g. every 30 - 300 seconds. The invention has recognized that the gain drifts are very slow compared to the physiologic motions. Thus, it is possible to average drift characterization data, e.g. the signal difference between diastolic and systolic phase in the cardiac trace, over long time periods to increase its accuracy.

In other words, during the measurement, such as MR data acquisition, the reference signal is continuously detected together with the desired physiologic components (e.g. respiration). The reference signal is normalized to yield a normalized preference signal, i.e. it is corrected based on the expected characteristic. In case of the cardiac trace, the difference between diastolic and systolic amplitude is determined.

Time-averaging of the normalized reference signal eliminates potential residual respiratory contamination and increases the signal quality. The time resolution should be small compared to physiologic variations (heartbeat duration and potentially respiratory duration), but high enough to follow the gain drift.

## Claims

1. A method for generating a movement signal (32) of an object (10), in particular a body part of a human or animal, wherein the movement signal provides quantitative information on a movement of the object (10), the method comprising the following steps:
a) Acquiring a navigation signal (108) from the object, wherein the navigation signal is an electromagnetic signal, which is modulated by movements of the object;
b) Extracting a reference signal (34) from the navigation signal;
c) Determining a parameter (45, 50) having a known time-dependency from the reference signal (34);
d) Correcting the navigation signal (108) based on the parameter or the time-average of said parameter in order to reduce a signal drift in the navigation signal (108);
e) Extracting the movement signal (32) from the navigation signal (108).

2. The method of claim 1, wherein the reference signal (34) has a quasi-periodic time dependency, and the parameter (45) is the amplitude of said quasi-periodic time dependency.

3. The method of claim 1 or 2, wherein the known time-dependency is that the parameter (45) is constant over time, or the time-average of said parameter is constant over time.

4. The method of one of the preceding claims, wherein the reference signal (34) is a cardiac signal related to the heart movement of the body of a human or animal, and the parameter (45) is the peak-to-peak signal amplitude between the point of maximum and minimum contraction of the heart, in particular averaged over several cycles of heart movement.

5. The method according to any one of the preceding claims, wherein the navigation signal (108) is acquired continuously over the duration of a medical examination, and the parameter or the time-average of said parameter of the reference signal (34) is determined repeatedly during the medical examination.

6. The method according to any one of the preceding claims, wherein the movement signal (32) provides quantitative information on the respiratory movement of the human or animal.

7. The method according to any one of the preceding claims, wherein the navigation signal (108) is acquired on several signal channels, in particular by a multi-channel RF coil (28) for magnetic resonance imaging.

8. The method according to any one of the preceding claims, wherein step e) is performed before step d), wherein the movement signal (32) is extracted from the navigation signal (108) before a correction thereof, and the correction based on the parameter or the time-average of said parameter is carried out on the extracted movement signal (32) to reduce a signal drift in the movement signal (32).

9. The method according to any one of the preceding claims, wherein the reference signal (34) and the movement signal (32) are extracted from the navigation signal (108) by a Principal Component Analysis or an Independent Component Analysis (122) and/or a frequency analysis.

10. The method according to any one of the preceding claims, wherein the navigation signal (108) is a Pilot Tone signal, wherein the Pilot Tone signal is generated by
• emitting a radio frequency signal (16) outside the bandwidth of an MR signal acquired during magnetic resonance imaging of the part of the human or animal body, and
• recording the radio frequency signal, modulated by a movement of the human or animal body, by an RF coil, in particular a multi-channel RF coil (28) for magnetic resonance imaging.

11. A method for generating a medical image data set of an object (10) undergoing a movement, in particular of a body part of a human or animal, the method comprising the following steps:
i. Placing the object (10) in a medical imaging device;
ii. Generating a movement signal (32) of the object by the method according to any one of claims 1 to 10, wherein the movement signal (32) provides quantitative information on a quasi-periodic movement of the object (10);
iii. Acquiring a medical image data set from the object (10), wherein the movement signal (32) is used for synchronizing or gating the acquisition of the medical image data set in order to reduce undesired motion artefacts in the medical image dataset.

12. The method according to claim 11, wherein the Medical imaging device (12) is an MR scanner and the medical image dataset is a Magnetic resonance image dataset.

13. The method according to claim 12, wherein the navigation signal (108) is a pilot tone signal recorded by a multi-channel RF coil, wherein the navigation signal is acquired on several signal channels, and wherein the channel combination corresponding to the reference signal (34) and/or the movement signal (32) is determined during a calibration phase before or at the beginning of the acquisition of the medical image data set, and is used in extracting the reference signal (34) and/or the movement signal (32) from the navigation signal (108).

14. Medical imaging device (12), in particular an MR scanner, comprising an RF coil and configured for performing the following steps:
i. Acquiring a navigation signal (108) from an object (10), in particular from a body part of a human or animal, by the RF coil, wherein the navigation signal is an electromagnetic signal, the amplitude of which is modulated by movements of the object (10);
ii. Extracting a reference signal (34) from the navigation signal,
iii. Determining a parameter having a known time-dependency from the reference signal (34);
iv. Correcting the navigation signal (108) based on the parameter or a time-average of said parameter;
v. Extracting a movement signal (32) from the navigation signal (108), wherein the movement signal (32) provides quantitative information on a quasi-periodic movement of the object (10);
vi. Acquiring a medical image data set from the object (10), wherein the movement signal (32) is used for synchronizing or gating (130) the acquisition of the medical image data set in order to reduce undesired motion artefacts in the medical image dataset.

15. Computer Program product comprising software code portions, which induce a processor (24), in particular a processor controlling a medical imaging device, to perform the method of one of claims 1 to 13, when the software code portions are executed on the processor.

## Patentansprüche

1. Verfahren zur Erzeugung eines Bewegungssignals (32) eines Objekts (10), insbesondere eines Körperteils eines Menschen oder eines Tiers, wobei das Bewegungssignal quantitative Informationen über eine Bewegung des Objekts (10) bereitstellt, wobei das Verfahren die folgenden Schritte umfasst:
a) Erfassen eines Navigationssignals (108) vom Objekt, wobei das Navigationssignal ein elektromagnetisches Signals ist, das durch Bewegungen des Objekts moduliert wird;
b) Extrahieren eines Referenzsignals (34) aus dem Navigationssignal;
c) Bestimmen eines Parameters (45, 50) mit einer bekannten Zeitabhängigkeit vom Referenzsignal (34);
d) Korrigieren des Navigationssignals (108) basierend auf dem Parameter oder dem Zeitmittel des Parameters, um eine Signaldrift im Navigationssignal (108) zu reduzieren;
e) Extrahieren des Bewegungssignals (32) aus dem Navigationssignal (108).

2. Verfahren nach Anspruch 1, wobei das Referenzsignal (34) eine quasiperiodische Zeitabhängigkeit aufweist, und der Parameter (45) die Amplitude der quasiperiodischen Zeitabhängigkeit ist.

3. Verfahren nach Anspruch 1 oder 2, wobei die bekannte Zeitabhängigkeit ist, dass der Parameter (45) im Zeitablauf konstant ist oder das Zeitmittel des Parameters im Zeitablauf konstant ist.

4. Verfahren nach einem der vorhergehenden Ansprüche, wobei das Referenzsignal (34) ein Herzsignal in Bezug auf die Herzbewegung des Körpers eines Menschen oder eines Tiers ist, und der Parameter (45) die Spitze-Spitze-Signalamplitude zwischen dem Punkt maximaler und minimaler Kontraktion des Herzens, insbesondere gemittelt über mehrere Herzbewegungszyklen, ist.

5. Verfahren nach einem der vorhergehenden Ansprüche, wobei das Navigationssignal (108) über die Dauer einer medizinischen Untersuchung kontinuierlich erfasst wird, und der Parameter oder das Zeitmittel des Parameters des Referenzsignals (34) während der medizinischen Untersuchung wiederholt bestimmt wird.

6. Verfahren nach einem der vorhergehenden Ansprüche, wobei das Bewegungssignal (32) quantitative Informationen über die Atembewegung des Menschen oder des Tiers bereitstellt.

7. Verfahren nach einem der vorhergehenden Ansprüche, wobei das Navigationssignal (108) auf mehreren Signalkanälen, insbesondere durch eine Mehrkanal-HF-Spule (28) für Magnetresonanz-Bildgebung, erfasst wird.

8. Verfahren nach einem der vorhergehenden Ansprüche, wobei Schritt e) vor Schritt d) ausgeführt wird, wobei das Bewegungssignal (32) aus dem Navigationssignal (108) vor der Korrektur davon extrahiert wird, und die Korrektur, die auf dem Parameter oder dem Zeitmittel des Parameters basiert, am extrahierten Bewegungssignal (32) durchgeführt wird, um eine Signaldrift des Bewegungssignals (32) zu reduzieren.

9. Verfahren nach einem der vorhergehenden Ansprüche, wobei das Referenzsignal (34) und das Bewegungssignal (32) durch eine Hauptkomponentenanalyse oder eine Analyse unabhängiger Komponenten (122) und/oder eine Frequenzanalyse aus dem Navigationssignal (108) extrahiert werden.

10. Verfahren nach einem der vorhergehenden Ansprüche, wobei das Navigationssignal (108) ein Pilottonsignal ist, wobei das Pilottonsignal erzeugt wird durch:
• Emittieren eine Hochfrequenzsignals (16) außerhalb der Bandbreite eines MR-Signals, das während einer Magnetresonanz-Bildgebung des Körperteils des Menschen oder des Tiers erfasst wird, und
• Aufzeichnen des durch eine Bewegung des Körpers des Menschen oder des Tiers modulierten Hochfrequenzsignals durch eine HF-Spule, insbesondere eine Mehrkanal-HF-Spule (28) für Magnetresonanz-Bildgebung.

11. Verfahren zur Erzeugung eines medizinischer Bilddatensatzes eines Objekts (10), das einer Bewegung unterworfen wird, insbesondere eines Körperteils eines Menschen oder eines Tiers, wobei das Verfahren die folgenden Schritte umfasst:
i. Anordnen des Objekts (10) in einer medizinischen Bildgebungsvorrichtung;
ii. Erzeugen eines Bewegungssignals (32) des Objekts durch das Verfahren nach einem der Ansprüche 1 bis 10, wobei das Bewegungssignal (32) quantitative Informationen über eine quasiperiodische Bewegung des Objekts (10) bereitstellt;
iii. Erfassen eines medizinischen Bilddatensatzes vom Objekt (10), wobei das Bewegungssignal (32) zum Synchronisieren oder Steuern der Erfassung des medizinischen Datensatzes verwendet wird, um unerwünschte Bewegungsartefakte im medizinischen Bilddatensatz zu reduzieren.

12. Verfahren nach Anspruch 11, wobei die medizinische Bildgebungsvorrichtung (12) ein MR-Scanner ist, und der medizinische Bilddatensatz ein Magnetresonanz-Bilddatensatz ist.

13. Verfahren nach Anspruch 12, wobei das Navigationssignal (108) ein Pilottonsignal ist, das von einer Mehrkanal-HF-Spule aufgezeichnet wird, wobei das Navigationssignal auf mehreren Signalkanälen erfasst wird, und wobei die Kanalkombination, die dem Referenzsignal (34) und/oder dem Bewegungssignal (32) entspricht, während einer Kalibrierphase vor oder zu Beginn der Erfassung des medizinischen Bilddatensatzes bestimmt und beim Extrahieren des Referenzsignals (34) und/oder des Bewegungssignals (32) aus dem Navigationssignal (108) verwendet wird.

14. Medizinische Bildgebungsvorrichtung (12), insbesondere MR-Scanner, umfassend eine HF-Spule und ausgelegt zum Ausführen der folgenden Schritte:
i. Erfassen eines Navigationssignals (108) von einem Objekt (10), insbesondere von einem Körperteil eines Menschen oder eines Tiers, durch die HF-Spule, wobei das Navigationssignal ein elektromagnetisches Signal ist, dessen Amplitude durch Bewegungen des Objekts (10) moduliert wird;
ii. Extrahieren eines Referenzsignals (34) aus dem Navigationssignal;
iii. Bestimmen eines Parameters mit einer bekannten Zeitabhängigkeit vom Referenzsignal (34);
iv. Korrigieren des Navigationssignals (108) basierend auf dem Parameter oder einem Zeitmittel des Parameters;
v. Extrahieren eines Bewegungssignals (32) aus dem Navigationssignal (108), wobei das Bewegungssignal (32) quantitative Informationen über eine quasiperiodische Bewegung des Objekts (10) bereitstellt;
vi. Erfassen eines medizinischen Bilddatensatzes vom Objekt (10), wobei das Bewegungssignal (32) zum Synchronisieren oder Steuern (130) der Erfassung des medizinischen Datensatzes verwendet wird, um unerwünschte Bewegungsartefakte im medizinischen Bilddatensatz zu reduzieren.

15. Computerprogrammprodukt, umfassend Softwarecodeabschnitte, die einen Prozessor (24), insbesondere einen Prozessor, der eine medizinische Bildgebungsvorrichtung steuert, zum Durchführen des Verfahrens nach einem der Ansprüche 1 bis 13 veranlasst, wenn die Softwarecodeabschnitte auf dem Prozessor ausgeführt werden.

## Revendications

1. Procédé de production d'un signal (32) de mouvement d'un objet (10), en particulier d'une partie du corps d'un humain ou d'un animal, dans lequel le signal de mouvement procure une information quantitative sur un mouvement de l'objet (10), le procédé comprenant les stades suivants :
a) on acquiert un signal (108) de navigation de l'objet, le signal de navigation étant un signal électromagnétique modulé par des mouvements de l'objet ;
b) on extrait un signal (34) de référence du signal de navigation ;
c) on détermine du signal (34) de référence un paramètre (45, 50) ayant une dépendance en fonction du temps, qui est connue ;
d) on corrige le signal (108) de navigation sur la base du paramètre ou de la moyenne dans le temps du paramètre, afin de réduire une dérive du signal (108) de navigation ;
e) on extrait le signal (32) de mouvement du signal (108) de navigation.

2. Procédé suivant la revendication 1, dans lequel le signal (34) de référence a une dépendance quasi périodique en fonction du temps et le paramètre (45) est l'amplitude de la dépendance quasi périodique en fonction du temps.

3. Procédé suivant la revendication 1 ou 2, dans lequel la dépendance en fonction du temps, qui est connue, est que le paramètre (45) est constant en fonction du temps ou que la moyenne dans le temps du paramètre est constante en fonction du temps.

4. Procédé suivant l'une des revendications précédentes, dans lequel le signal (34) de référence est un signal cardiaque se rapport au mouvement du cœur du corps d'un humain ou d'un animal, et le paramètre (45) est une amplitude de signal de crête à crête entre le point de contraction maximum et minimum du cœur, en particulier dont on a fait la moyenne sur plusieurs cycles de mouvement du cœur.

5. Procédé suivant l'une quelconque des revendications précédentes, dans lequel on acquiert le signal (108) de navigation continuellement pendant la durée d'un examen médical, et on détermine de manière répétée pendant l'examen médical le paramètre ou la moyenne en fonction du temps du paramètre du signal (34) de référence.

6. Procédé suivant l'une quelconque des revendications précédentes, dans lequel le signal (32) de mouvement procure une information quantitative sur le mouvement respiratoire de l'humain ou de l'animal.

7. Procédé suivant l'une quelconque des revendications précédentes, dans lequel on acquiert le signal (108) de navigation sur plusieurs canaux de signal, en particulier par une bobine (28) RF à plusieurs canaux pour l'imagerie par résonnance magnétique.

8. Procédé suivant l'une quelconque de revendications précédentes, dans lequel on effectue le stade e) avant le stade d), dans lequel on extrait le signal (32) de mouvement du signal (108) de navigation avant sa correction et on effectue la correction sur la base du paramètre ou de la moyenne dans le temps du paramètre sur le signal (32) de mouvement, qui a été extrait, afin de réduire une dérive du signal (32) de mouvement.

9. Procédé suivant l'une quelconque des revendications précédentes, dans lequel on extrait le signal (34) de référence et le signal (32) de mouvement du signal (108) de navigation par une Principal Component Analysis ou par une Independent Component Analysis (122) et/ou par une analyse de fréquence.

10. Procédé suivant l'une quelconque des revendications précédentes, dans lequel le signal (108) de navigation est un signal Pilot Tone, le signal Pilot Tone étant produit en
• en émettant un signal (16) fréquence radio à l'extérieur de la largeur de bande d'un signal RM acquis pendant une imagerie par résonance magnétique de la partie du corps humain ou animal, et
• en enregistrant le signal de fréquence radio modulé par un mouvement du corps humain ou animal par une bobine RF, en particulier une bobine (28) RF à plusieurs canaux pour l'imagerie par résonance magnétique.

11. Procédé de production d'un ensemble de données d'image médicale d'un objet (10) subissant un mouvement, en particulier d'une partie du corps d'un humain ou d'un animal, le procédé comprenant les stades suivants :
i. on met l'objet (10) dans un dispositif d'imagerie médicale ;
ii. on produit un signal (32) de mouvement de l'objet par le procédé suivant l'une quelconque des revendications 1 à 10, le signal (32) de mouvement procurant une information quantitative sur un mouvement quasi périodique de l'objet (10) ;
iii. on acquiert un ensemble de données d'image médicale de l'objet (10), le signal (32) de mouvement étant utilisé pour synchroniser ou moduler l'acquisition de l'ensemble de données d'image médicale, afin de réduire des artéfacts de mouvement non souhaités dans le jeu de données d'image médicale.

12. Procédé suivant la revendication 11, dans lequel le dispositif (12) d'imagerie médicale est un scanner RM et le jeu de données d'image médicale est un jeu de données d'image de résonnance magnétique.

13. Procédé suivant la revendication 12, dans lequel le signal (108) de navigation est un signal pilot tone enregistré par une bobine RF à plusieurs canaux, le signal de navigation étant acquis sur plusieurs canaux de signal et la combinaison de canaux correspondant au signal (34) de référence et/ou le signal (32) de mouvement étant déterminé pendant une phase d'étalonnage avant ou au début de l'acquisition de l'ensemble de données d'image médicale et étant utilisé dans l'extraction du signal (34) de référence et/ou du signal (32) de mouvement du signal (108) de navigation.

14. Dispositif (12) d'imagerie médicale, en particulier scanner RM, comprenant une bobine RF et configuré pour effectuer les stades suivants :
i. on acquiert un signal (108) de navigation de l'objet, en particulier d'une part du corps d'un humain ou d'un animal, par la bobine RF, le signal de navigation étant un signal électromagnétique, dont l'amplitude est modulée par des mouvements de l'objet ;
ii. on extrait un signal (34) de référence du signal de navigation ;
iii. on détermine du signal (34) de référence un paramètre (45, 50) ayant une dépendance en fonction du temps, qui est connue ;
iv. on corrige le signal (108) de navigation sur la base du paramètre ou de la moyenne dans le temps du paramètre, afin de réduire une dérive du signal (108) de navigation ;
v. on extrait un signal (32) de mouvement du signal (108) de navigation, le signal (32) de mouvement procurant une information quantitative sur un mouvement quasi périodique de l'objet ( 10) ;
vi. on acquiert un ensemble de données d'image médicale de l'objet (10), le signal (32) de mouvement étant utilisé pour la synchronisation ou la modulation (130) de l'acquisition de l'ensemble de données d'image médicale, afin de réduire des artefacts de mouvement non souhaités dans le jeu de donnée d'image médicale.

15. Produit de programme d'ordinateur, comprenant des parties de code logiciel, qui induisent un processeur (24), en particulier un processeur commandant un dispositif d'imagerie médicale, à effectuer le procédé suivant l'une des revendications 1 à 13, lorsque les parties de code de logiciel sont exécutées sur le processeur.
